# FASCICULE DE BREVET EUROPEEN

(11) **EP 0 592 586 B1**
(45) Date de publication et mention de la délivrance du brevet: **27.08.1997**
(21) Numéro de dépôt: 92915287.4
(22) Date de dépôt: 02.07.1992
(51) Int. Cl.: C08L 89/06, D01C 3/00

(54) **UTILISATION DE LA PEAU NON PIGMENTEE DE POISSON, EN PARTICULIER DE POISSON PLAT COMME NOUVELLE SOURCE INDUSTRIELLE DE COLLAGENE, PROCEDE D'EXTRACTION, COLLAGENE ET BIOMATERIAU AINSI OBTENUS**
VERWENDUNG DER NICHTPIGMENTIERTEN FISCHHAUT, INSBESONDERE VON PLATTFISCHEN ALS INDUSTRIELLE HERKUNFT FÜR KOLLAGEN. VERFAHREN ZUR EXTRAHIERUNG, KOLLAGEN UND BIOMATERIAL SO ERHALTEN.
USE OF UNPIGMENTED FISH SKIN, PARTICULARLY FROM FLAT FISH, AS A NOVEL INDUSTRIAL SOURCE OF COLLAGEN, EXTRACTION METHOD, AND COLLAGEN AND BIOMATERIAL THEREBY OBTAINED

(30) Priorité: 04.07.1991 FR 9108400
(43) Date de publication de la demande: 20.04.1994
(73) Titulaire: COLETICA, 69007 Lyon (FR)
(72) Inventeur: DEVICTOR, Pierre, F-69350 La Mulatière (FR); ALLARD, Roland, F-69230 St.-Genis-Laval (FR); PERRIER, Eric, F-38200 Vienne (FR); HUC, Alain, F-69110 Ste-Foy-Les-Lyon (FR)
(74) Mandataire: Portal, Gérard
(86) Numéro de dépôt international: FR9200621
(87) Numéro de publication internationale: WO9301241

(56) Documents cités:
- WO-A-89/11799
- CH-A- 229 190
- DE-C- 736 769
- FR-A- 582 543
- FR-A- 582 544
- FR-A- 647 030
- JP-A-62 083 849
- CHEMICAL ABSTRACTS, vol. 107, no. 17, 26 Octobre 1987, Columbus, Ohio, US; abstract no. 150796f, page 382 ; & JP-A-62 083 849
- INDIAN JOURNAL OF EXPERIMENTAL BIOLOGY vol. 18, no. 7, 1 Juillet 1980, NEW DELHI pages 733 - 735 S.C.GANTAYAT ET AL. 'COLLAGEN CONTENT OF SKIN OF FRESH WATER TELEOST'

## Description

La présente invention concerne essentiellement l'utilisation de la peau non pigmentée de poisson, en particulier de poisson plat, comme nouvelle source industrielle de collagène, un procédé d'extraction de collagène, ainsi que du collagène et un biomatériau ainsi obtenus.

Le collagène protéine de soutien des tissus conjonctifs de l'être vivant trouve des applications de plus en plus larges dans les domaines cosmétiques et pharmaceutiques.

Les propriétés principales sont les suivantes :
- bonnes propriétés mécaniques,
- action sur le développement cellulaire et par là sur la régénération tissulaire,
- pouvoir hémostatique,
- très bonne biocompatibilité,
- biodégradabilité,
- support naturel conférant une meilleure biodisponibilité à de nombreux actifs.

Cette protéine, du fait de ses remarquables qualités a conduit à la réalisation de nombreux biomatériaux dont le marché est en expansion continue.

Parmi les nombreuses préparations à base de collagène, les principales paraissent être les suivantes :
- solution pour les produits cosmétiques,
- solution pour la réalisation de peaux artificielles,
- éponge hémostatique,
- pansement cicatrisant,
- matériau de reconstruction osseuse,
- capsules por la protection d'acitfs et l'amélioration de leur biodisponibilité,
- biomatériaux sous forme solide ou liquide pour le comblement des tissus mous.

A la lecture de la liste précédente qui est loin d'être exhaustive, il est possible de se rendre compte que le collagène va devenir une substance d'une grande importance industrielle.

Le développement des matériaux collagéniques s'effectuera d'autant plus rapidement que leur coût sera plus faible.

C'est pourquoi les inventeurs ont cherché exploiter de nouvelle sources de collagène permettant de remplir les procédés industriels, d'améliorer les rendements, et par là même d'abaisser les coûts de préparation de la protéine.

A l'heure actuelle, le principal tissu utilisé pour l'obtention de collagène est la peau de veau.

L'exploitation de cette matière première se heurte à deux difficultés.

D'une part, la collecte de la peau ne s'effectue pas en général dans des conditions sanitaires parfaites, ce qui entraîne l'obligation d'utiliser la peau dans les plus brefs délais après l'abattage de l'animal.

D'autre part, un important traitement de la peau est nécessaire pour éliminer les poils et les tissus sous-cutanés. Ce travail est réalisé grâce à des installations de tannerie impliquant la construction d'un atelier indépendant du site de transformation du collagène en biomatériau. Une telle infrastructure nécessite des investissements relativement lourds qui se répercuteront sur le coût du collagène obtenu.

De plus, la peau de veau bien qu'étant un tissu d'animal jeune fournit des quantités de collagène acido soluble relativement faibles, environ 12 g de protéine sèche pour 1 kilo de peau fraîche.

Il a été proposé dans le document JP-A-62-83 849 = CA 107: 150796 ou dans WO 89/11799 la production de collagène à partir de divers tissus d'animaux, incluant la peau de poisson. Dans WO 89/11799 on produit des fibres de collagène qui sont ensuite utilisées comme agent de texture dans des produits alimentaires.

Pour éviter en grande partie le traitement de la peau et pour obtenir de meilleurs rendements d'extraction, les inventeurs ont eu l'idée d'utiliser la peau non pigmentée de poisson.

Il a en effet été observé par les inventeurs que la peau de poisson pigmentée n'est pas utilisable car le pigment est très difficilement éliminé au cours du procédé de préparation du collagène. La protéine ainsi obtenue conduit alors à des biomatériaux pigmentés impurs.

De ce fait, les inventeurs se sont tournés vers des poissons dont la peau n'est pas pigmentée et qui est disponible en grande quantité. Les espèces entrant dans cette catégorie sont les poissons plats, en particulier ceux qui sont pêchés de façon industrielle comme par exemple la sole, la limande, le turbot, barbue, et dont la préparation des filets implique un dépeçage. La peau ventrale de ces poissons est non pigmentée, elle est disponible écaillée en quantités importantes.

Ainsi, selon un premier aspect, la présente invention concerne l'utilisation de la peau non pigmentée de poisson obtenue après dépeçage des peaux en excluant les peaux pigmentées pour obtenir du collagène. De préférence, cette peau non pigmentée est une peau de poisson plat, en particulier une peau ventrale. Cette peau non pigmentée est avantageusement obtenue après dépeçage du poisson frais qui est congelé immédiatement après le dépeçage, ce qui garantit une très bonne qualité de la matière première tant sur le plan bactériologique que sur celui du caractère natif de la protéine.

Par ailleurs, un avantage inattendu réside dans le fait que la peau ainsi récupérée et congelée est utilisable immédiatement pour l'extraction de collagène sans travail de tannerie préalable. Egalement, il s'est avéré que la quantité de collagène extraite partir de cette matière première est environ trois fois supérieure à celle obtenue à partir de la peau de veau.

De ce fait, la mise en oeuvre de cette nouvelle source de collagène conduira à l'obtention d'une protéine d'un coût moins élevé avec des propriétés natives préservées de façon reproductible.

Selon un deuxième aspect, la présente invention concerne aussi un procédé d'extraction de collagène, caractérisé en ce qu'on utilise de la peau non pigmentée de poisson obtenue après dépeçage des peaux de poisson en excluant les peaux pigmentées, que l'on soumet à une extraction de collagène que l'on récupère. Avantageusement, ledit collagène est natif.

Selon une variante de réalisation de ce procédé, la peau non pigmentée est une peau de poisson plat, en particulier une peau ventrale. Avantageusement, cette peau est une peau obtenue après dépeçage du poisson frais qui est congelé immédiatement après dépeçage.

Cette peau est également utilisée pour l'extraction de collagène sans travail de tannerie préalable.

Selon une variante de réalisation particulière, on utilise de la peau non pigmentée de poisson plat pêché de façon industrielle tel que par exemple la sole, la limande, le turbot, le barbue.

Selon une variante de réalisation particulière, on extrait du collagène acido soluble à l'état natif par extraction dans une solution acide, comme cela est bien connu à l'homme de l'art. On sépare ensuite le collagène natif par précipitation à partir du surnageant, par addition d'un agent de précipitation, par exemple du chlorure de sodium. Si cela est souhaité, on peut procéder à une déréticulation de ce collagène natif.

Selon une autre variante de réalisation particulière, on fabrique un biomatériau, en totalité ou en partie, avec ce collagène natif.

L'invention sera maintenant décrite en référence à un exemple d'extraction de collagène mettant en oeuvre le procédé d'extraction selon l'invention, donné simplement à titre d'illustration et qui ne saurait donc en aucune façon limiter la portée de l'invention.

### Exemple

### Préparation de collagène acido soluble à partir de 40 kg de peaux de sole congelées

### 1. Broyage de la peau

Les peaux ventrales de sole pêchées industriellement sont livrées congelées en blocs de 20 kg. Ceux-ci sont découpés en blocs de 2 kg à l'aide d'une scie à ruban. Les morceaux récupérés sont alors broyés et décongelés grâce à un broyeur à couteau muni d'une cuve étanche de 40 l. 39 kg de broyat sont obtenus.

### 2. Lavage du broyat

Le broyat est lavé par deux bains successifs de tampon phosphate (21,7 g/l NA₂ HPO₄ et 0,78 g/l de KH₂ PO₄). La concentration en broyat étant de 1 kg pour 5 l de bain et le temps de contact de 1 h. Le broyat est séparé du tampon par centrifugation grâce à une décanteuse en continu tournant à 4 000 tr/min. Le phosphate est ensuite éliminé dans les mêmes conditions par deux lavages successifs à l'eau permutée stérile.

### 3. Extraction acide

Le broyat obtenu est alors placé dans une solution d'acide acétique 0,25 M à une concentration de 1 kg pour 10 l de bain. L'ensemble est agité pendant 15 min et le surnageant est récupéré par centrifugation en continu dans les mêmes conditions que précédemment.

Cette opération d'extraction est réalisée deux fois. Les surnageants sont réunis et le collagène qu'ils renferment est précipité par addition de chlorure de sodium à une concentration de 7 %. Les fibres obtenues sont dialysées contre l'eau permutée jusqu'à élimination complète des sels. Le collagène obtenu peut être soit remis en solution acide, soit lyophilisé.

A partir de 40 kg de peaux de sole fraîche, 1,6 kg de collagène sec sosnt obtenus. Le rendement d'extraction est plus de trois fois supérieur à celui de la peau de veau.

Voici les caractéristiques d'une solution de collagène acido soluble de peau de sole dans un milieu à 0,1 % d'acide sorbique.

### Analyses chimiques

| | |
|---|---|
| Matières sèches | 0,46 |
| Matières minérales | 0,08 |
| Azote total | 0,075 |
| Protéines totales (d'après azote) | 0,40 |
| Hydroxyproline | 0,03 |
| Collagène d'après hydroxyproline | 0,40 |
| Acidité (en acide sorbique) | 0,10 |

### Analyses physiques

- Electrophorèse
   - sous unités β (200 000 daltons) 56 %
      α (100 000 daltons) 44 %
   - pas de sous-unités inférieures à 100 000 daltons.
- Calorimétrie différentielle programmée :
   - température de début de dénaturation en °C : 28
   - température de fin de dénaturation en °C : 37
   - enthalpie de dénaturation (J/mg collagène) 5 x 10⁻²
- Diffraction des rayons X
   - présence de la structure hélicoïdale.

Il a également été déterminé la composition en acides aminés du collagène acido soluble obtenu à l'exemple précédent et qui a été extrait de la peau ventrale de sole.

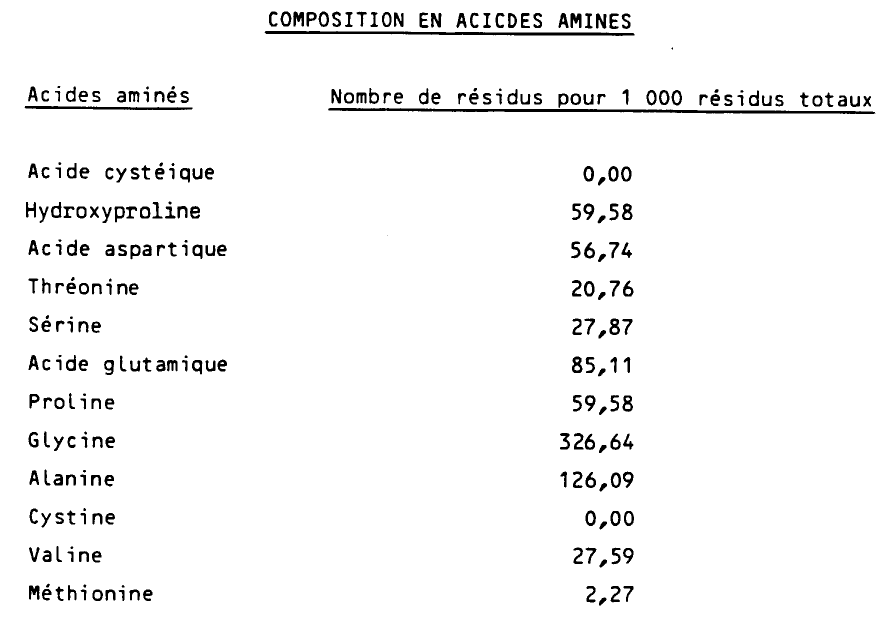

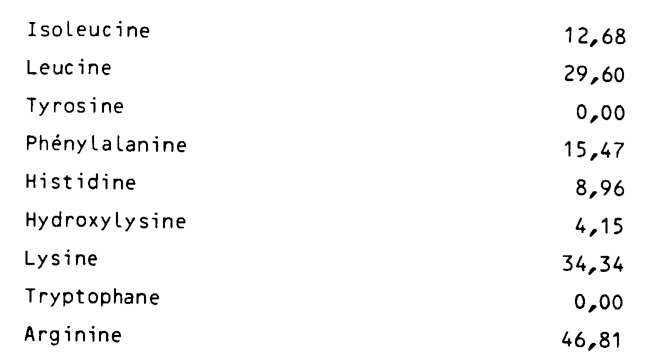

## Revendications

1. Utilisation de la peau non pigmentée de poisson obtenue après dépeçage des peaux de poisson en excluant les peaux pigmentées pour obtenir du collagène.

2. Procédé d'extraction de collagène, caractérisé en ce qu'on utilise de la peau non pigmentée de poisson obtenue après dépeçage des peaux de poisson en excluant les peaux pigmentées, que l'on soumet à une extraction de collagène que l'on récupère.

3. Procédé selon la revendication 2, caractérisé en ce que ledit collagène est natif.

4. Procédé selon la revendication 2 ou 3, caractérisé en ce qu'on utilise de la peau de poisson plat.

5. Procédé selon l'une des revendications 2 à 4, caractérisé en ce qu'on utilise de la peau ventrale non pigmentée de poisson.

6. Procédé selon l'une des revendications 2 à 5, caractérisé en ce que la peau est obtenue après dépeçage du poisson frais et qui est congelée immédiatement après dépeçage.

7. Procédé selon l'une des revendications 2 à 6, caractérisé en ce que le poisson est choisi parmi la sole, la limande, le turbot, le barbue.

8. Procédé selon l'une des revendications 2 à 7, caractérisé en ce qu'on extrait le collagène acido-soluble par extraction dans une solution acide et on effectue une séparation du collagène par précipitation à partir du surnageant.

9. Procédé selon l'une des revendications 2 à 8, caractérisé en ce que la peau de poisson non pigmentée est tout d'abord broyée puis est soumise à un lavage avec une solution de tampon phosphate, la solution tampon phosphate est séparée du matériau broyé et le broyat est lavé avec de l'eau permutée stérile pour éliminer le phosphate avant l'extraction du collagène.

10. Procédé selon la revendication 8 ou 9, caractérisé en ce que l'on sépare le collagène natif par précipitation à partir du surnageant, par addition d'un agent de précipitation, et en obtenant ainsi des fibres de collagène natif.

11. Procédé selon la revendication 10, caractérisé en ce que l'agent de précipitation est du chlorure de sodium.

12. Procédé selon la revendication 10 ou 11, caractérisé en ce que les fibres sont soumises à une dialyse contre de l'eau permutée jusqu'à l'élimination complète des sels pour donner un collagène natif qui est récupéré.

13. Procédé selon l'une des revendications précédentes, caractérisé en ce que le collagène récupéré est lyophilisé.

14. Procédé selon l'une des revendications 8 à 13, caractérisé en ce que le collagène natif présente 56 % de sous unité β à 200 000 daltons, 44 % de sous unité α à 100 000 daltons et ne contient pas de sous unité inférieure à 100 000 dalton comme mesuré par l'électrophorèse ; et présente une structure hélicoïdale.

15. Procédé selon l'une des revendications précédentes, caractérisé en ce qu'on procède à une déréticulation du collagène natif.

16. Procédé selon l'une des revendications précédentes, caractérisé en ce qu'on fabrique un biomatériau, en totalité ou en partie, avec ce collagène natif.

## Patentansprüche

1. Verwendung nicht-pigmentierter Fischhaut, die nach dem Zerlegen von Fischhaut erhalten wird, wobei pigmentierte Haut ausgeschlossen wird, um Kollagen zu erhalten.

2. Verfahren zur Extraktion von Kollagen, dadurch gekennzeichnet, daß nicht-pigmentierte Fischhaut verwendet wird, die nach dem Zerlegen von Fischhaut erhalten wird, wobei pigmentierte Haut ausgeschlossen wird, und die einer Extraktion von Kollagen unterworfen wird, das gewonnen wird.

3. Verfahren nach Anspruch 2, dadurch gekennzeichnet, daß das Kollagen nativ ist.

4. Verfahren nach Anspruch 2 oder 3, dadurch gekennzeichnet, daß Haut von Plattfisch verwendet wird.

5. Verfahren nach einem der Ansprüche 2 bis 4, dadurch gekennzeichnet, daß nicht-pigmentierte ventrale Fischhaut verwendet wird.

6. Verfahren nach einem der Ansprüche 2 bis 5, dadurch gekennzeichnet, daß die Haut nach dem Zerlegen von frischem Fisch erhalten wird, und unmittelbar nach dem Zerlegen tiefgefroren wird.

7. Verfahren nach einem der Ansprüche 2 bis 6, dadurch gekennzeichnet, daß der Fisch aus Seezunge, Rotzunge, Steinbutt, Tarbutt ausgewählt wird.

8. Verfahren nach einem der Ansprüche 2 bis 7, dadurch gekennzeichnet, daß das säurelösliche Kollagen durch die Extraktion in einer sauren Lösung extrahiert wird, und eine Trennung des Kollagens durch Ausfällung aus dem Überstand durchgeführt wird.

9. Verfahren nach einem der Ansprüche 2 bis 8, dadurch gekennzeichnet, daß die nicht-pigmentierte Fischhaut zuerst zerkleinert wird, dann einer Wäsche mit einer Phosphatpuffer-Lösung unterworfen wird, die Phosphatpuffer-Lösung vom zerkleinerten Material getrennt wird, und das zerkleinerte Material mit sterilem permutierten Wasser gewaschen wird, um das Phosphat vor der Extraktion des Kollagens zu eliminieren.

10. Verfahren nach Anspruch 8 oder 9, dadurch gekennzeichnet, daß das native Kollagen durch Ausfällung aus dem Überstand und durch den Zusatz eines Fällungsmittels abgetrennt wird, wobei so native Kollagenfasern erhalten werden.

11. Verfahren nach Anspruch 10, dadurch gekennzeichnet, daß das Fällungsmittel Natriumchlorid ist.

12. Verfahren nach Anspruch 10 oder 11, dadurch gekennzeichnet, daß die Fasern einer Dialyse gegen permutiertes Wasser bis zur vollständigen Eliminierung von Salzen unterworfen werden, wobei ein natives Kollagen erhalten wird, das gewonnen wird.

13. Verfahren nach einem der vorhergehenden Ansprüche, dadurch gekennzeichnet, daß das gewonnene Kollagen lyophilisiert wird.

14. Verfahren nach einem der Ansprüche 8 bis 13, dadurch gekennzeichnet, daß das native Kollagen 56 % β-Untereinheiten mit 200 000 Dalton, 44 % α-Untereinheiten mit 100 000 Dalton aufweist und keine Untereinheiten mit weniger als 100 000 Dalton enthält, wie durch Elektrophorese gemessen; und eine Helix-Struktur hat.

15. Verfahren nach einem der vorhergehenden Ansprüche, dadurch gekennzeichnet, daß eine Entnetzung des nativen Kollagens durchgeführt wird.

16. Verfahren nach einem der vorhergehenden Ansprüche, dadurch gekennzeichnet, daß ein Biomaterial gänzlich oder teilweise mit nativem Kollagen hergestellt wird.

## Claims

1. Use of unpigmented fish skin obtained after cutting the fish skins by excluding the pigmented skins in order to obtain collagen.

2. Method for extracting collagen, characterised in that it uses unpigmented fish skin obtained after cutting the fish skins by excluding the pigmented skins, which skin is subjected to a collagen extraction which is recovered.

3. Method according to claim 2, characterised in that said collagen is a native collagen.

4. Method according to claim 2 or 3, characterised in that it uses flat fish skin.

5. Method according to one of claims 2 to 4, characterised in that it uses unpigmented ventral fish skin.

6. Method according to one of claims 2 to 5, characterised in that the skin is obtained after cutting the fresh fish and which skin is frozen immediately after cutting.

7. Method according to one of claims 2 to 6, characterised in that the fish is selected from sole, dab, turbot, brill.

8. Method according to one of claims 2 to 7, characterised in that the acid-soluble collagen is extracted by extraction in an acid solution and separated by precipitation from the supernatant.

9. Method according to one of claims 2 to 8, characterised in that the unpigmented fish skin is firstly ground and then subjected to washing with a phosphate buffer solution, the phosphate buffer solution is separated from the ground material and the ground material is washed with deionised sterilised water in order to eliminate the phosphate before the extraction of collagen.

10. Method according to claim 8 or 9, characterised in that the native collagen is separated by precipitation from the supernatant, by addition of a precipitating agent, and thus obtaining fibers from the native collagen.

11. Method according to claim 10, characterised in that the precipitating agent is sodium chloride.

12. Method according to claim 10 or 11, characterised in that the fibers are subjected to dialysis against deionised water until complete elimination of the salts in order to give a native collagen which is recovered.

13. Method according to one of the preceding claims, characterised in that the recovered collagen is lyophilised.

14. Method according to one of claims 8 to 13, characterised in that the native collagen has 56% of subunits β of 200,000 dalton, 44% of subunits α of 100,000 dalton, and does not have subunits lower than 100,000 dalton, as measured by electrophoresis and has a helicoidal structure.

15. Method according to one of the preceding claims, characterised in that the native collagen is decrosslinked.

16. Method according to one of the preceding claims, characterised in that a biomaterial is produced totally or partially with such native collagen.
